# EUROPÄISCHE PATENTANMELDUNG

(11) **EP 1 450 121 A2**
(43) Veröffentlichungstag der Anmeldung: **25.08.2004**
(21) Anmeldenummer: 03022752.4
(22) Anmeldetag: 09.10.2003
(51) Int. Cl.: F25D 25/00, C12M 1/00

(54) **Klimaschrank und insbesondere Klimakühlschrank**

(30) Priorität: 30.01.2003 DE 10303736
(71) Anmelder: Kendro Laboratory Products GmbH, 63505 Langenselbold (DE)
(72) Erfinder: Melching, Achim, 63505 Langenselbold (DE); Brömsen, Olaf, 64546 Mörfelden-Walldorf (DE); Bidlingmaier, Dieter, 63486 Bruchköbel (DE); Stahl, Hermann, 61130 Nidderau-Ostheim (DE)
(74) Vertreter: Tomerius, Isabel, Dr.

(57) **Zusammenfassung**

Die Erfindung betrifft einen Klimaschrank (1), insbesondere einen Klimakühlschrank, dessen Innenraum (2) mit Objektträgern (6) mit Hilfe einer Transportvorrichtung (7) be- oder entladen wird. Be- und Entladen erfolgen durch eine mit einer Tür (9) verschließbaren Ladeöffnung (8), die in ihrer Größe an die Abmessungen der Objektträger (6) angepasst und in einer Seitenwand des Klimaschranks (1) angeordnet ist. Der Klimaschrank (1) weist außerdem eine Gaszufuhrvorrichtung auf, deren wenigstens eine Gasausblasöffnung (10) so angeordnet ist, dass der Querschnitt der Ladeöffnung (8) im Bereich der Gasausblasöffnungen (10) bei Zufuhr des Gases von einem Gasvorhang abgedeckt wird.

## Beschreibung

Die Erfindung betrifft einen Klimaschrank und insbesondere einen Klimakühlschrank. Klimaschränke werden üblicherweise dazu verwendet, Objekte unter definierten klimatischen Bedingungen wie bei einer definierten Temperatur oder in einer Atmosphäre definierter Zusammensetzung aufzubewahren. Beispiele für Klimaschränke sind Wärmeschränke, Kühlschränke oder Inkubatoren, in denen Zellkulturen oder Mikroorganismen bei erhöhter Temperatur und sehr hoher Luftfeuchtigkeit und gegebenenfalls in einer mit Kohlendioxid angereicherten Atmosphäre über längere Zeit gelagert werden. Die Objekte sind im Allgemeinen auf geeigneten Objektträger aufgebracht, wie zum Beispiel Mikrotiterplatten. Zur Aufnahme der Objektträger befinden sich im klimatisierten Innenraum des Klimaschrankes üblicherweise ein oder mehrere Haltegestellte. Die Haltegestelle sind entweder im Innenraum feststehend oder drehbar gelagert (sogenanntes Karussell).

Der Innenraum des Klimaschranks ist in der Regel über eine Tür zugänglich, die groß genug ist, um ein Haltegestell im Innenraum zu postieren und es aus dem Innenraum zu entnehmen. Außerdem kann der Innenraum durch die Tür gereinigt und gewartet werden.

Die Beschickung des Haltegestells mit Objektträgern erfolgt vorzugsweise automatisch mit Hilfe einer geeigneten Transportvorrichtung. Da das Öffnen der großen Haupttür zu empfindlichen Störungen der klimatischen Verhältnisse im Innenraum führen würde, ist vorgeschlagen worden, für das Be- und Entladen der Objektträger eine gesonderte Schleuse zu verwenden, die einen möglichst geringen Durchgangsquerschnitt aufweist und nur gerade groß genug ist, um die Objektträger mit Hilfe der Transportvorrichtung in den Innenraum und aus diesem heraus zu bringen. Da für den Hin- und Hertransport der Objektträger nur eine sehr kleine Öffnung in der Außenwand des Klimaschranks freigegeben werden muss, sind die Auswirkungen auf die Klimabedingungen im Innenraum deutlich geringer, und ein kontinuierlicher Betrieb des Klimaschranks ohne gravierende Störungen des Klimas im Innenraum wird möglich.

Eine derartige Vorrichtung ist beispielsweise in der WO 98/05753 A1 beschrieben. Der dort beschriebene Klimaschrank weist neben einer Haupttür in der gegenüberliegenden, rückseitigen Wand eine weitere, kleinere Öffnung auf, die zum Hinein- und Heraustransportieren von Objektträgern in den Innenraum dient. Die kleinere, rückwärtige Öffnung, die dort als Fensteröffnung bezeichnet ist, ist mit einer Tür verschließbar. Als Haltegestell für die Objektträger im Inneren des Klimaschranks dienen ein oder mehrere Karusselle.

Durch die Verwendung der verkleinerten Fensteröffnung beim Transport der Objektträger in die Klimakammer hinein oder aus dieser heraus können die Einwirkungen der Außenatmosphäre auf den Innenraum deutlich reduziert, aber nicht vollständig unterbunden werden. So kommt es auch beim Öffnen des kleinen Fensters zu einem gewissen Austausch der Atmosphäre und zu einer Beeinflussung der Temperatur. Üblicherweise ist dies nicht weiter störend. In einigen besonderen Fällen kann es jedoch zu einer Beeinträchtigung des Klimas im Innenraum des Klimaschranks kommen. Wird zum Beispiel der Klimaschrank als Klimakühlschrank verwendet, wird bei jedem Hineintransportieren eines Objektträgers eine geringe Menge an Feuchtigkeit aus der Umgebungsatmosphäre in den Innenraum der Klimakammer eingeschleppt. Die Feuchtigkeit sammelt sich allmählich im Innenraum an und kondensiert oder friert wegen der verminderten Temperatur im Innenraum aus. Wenn die Feuchtigkeit an den Objektträgern kondensiert oder ausfriert, kann dies zu einer Schädigung der Mikroorganismen oder Zellkulturen und zu einer Verunreinigung der Objekte führen.

In der US 6,467,285 B2 ist ein Klimakühlschrank beschrieben, welcher der Vorrichtung der WO 98/05753 A1 sehr ähnlich ist. Der Klimakühlschrank weist in seinem Innenraum ein Karussell für die Aufnahme von Objektträgern auf, welche mit einer Transportvorrichtung automatisch in den Innenraum transportiert und aus diesem wieder herausbefördert werden. Auch hier erfolgt der Transport nicht durch die Haupttür des Klimaschrankes, sondern durch eine kleinere Transportschleuse. Anders als in der WO 98/05753 A1 ist hier jedoch die Schleuse als eine sowohl zum Innenraum des Klimaschranks als auch zu dessen Außenseite hin beidseitig verschließbare Kammer ausgebildet, die für sich allein klimatisiert werden kann. Um einen Objektträger in den Innenraum der Vorrichtung zu schaffen, wird zunächst die äußere Tür der Schleusenkammer geöffnet und der Objektträger in die Schleusenkammer geschoben, während die innere Tür der Schleusenkammer geschlossen ist. Dann wird auch die äußere Tür der Schleusenkammer verschlossen, und in die Schleusenkammer wird aus einer einzigen Zuleitung, die in einen oberen, innenseitigen Bereich der Kammer mündet, ein trockenes Gas eingeleitet, um die Feuchtigkeit aus der Schleusenkammer zu verdrängen und diese abzukühlen. Anschließend wird die innere Tür der Schleusenkammer geöffnet, und der Objektträger wird aus der Schleusenkammer zum Karussell transportiert und dort abgelegt. Zum Heraustransportieren des Objektträgers aus dem Klimakühlschrank werden die Schritte in umgekehrter Reihenfolge durchgeführt. Durch die beschriebenen Maßnahmen wird die Beeinflussung des Klimas im Innenraum des Klimakühlschranks durch die Außenatmosphäre noch weiter reduziert als im Falle der WO 98/05753 A1. Die Vorrichtung ist jedoch recht kompliziert aufgebaut, und der Transport eines jeden Objektträgers dauert relativ lange, da der Objektträger eine längere Zeit in der Schleusenkammer verbringen muss, bis das trockene Gas die Atmosphäre in der Schleusenkammer vollständig ersetzt hat.

Es bestand daher nach wie vor ein Bedarf an einem Klimaschrank, insbesondere nach einem Klimakühlschrank, welcher einfach aufgebaut ist und dabei dennoch zuverlässig eine Beeinträchtigung der Klimabedingungen im Innenraum durch Einwirkungen der Außenatmosphäre verhindert. **Aufgabe** der Erfindung ist es entsprechend, eine derartige Vorrichtung anzugeben.

Die Lösung dieser Aufgabe gelingt mit dem Klimaschrank gemäß Anspruch 1. Weiterbildungen und bevorzugte Ausführungsformen sind den Unteransprüchen zu entnehmen.

Die Erfindung betrifft also einen Klimaschrank der eingangs beschriebenen Art, bei welchem der Transport der Objektträger durch eine gesonderte Ladeöffnung erfolgt, die in ihrer Größe den Abmessungen der Objektträger angepasst ist. Die Ladeöffnung ist also nur so groß wie nötig, um einen Objektträger mittels der dafür vorgesehenen Transportvorrichtung in den Innenraum oder aus diesem heraus zu befördern. Der erfindungsgemäße Klimaschrank kann also grundsätzlich wie die im Stand der Technik bekannten Klimaschränke ausgebildet sein und unterscheidet sich von diesen im Wesentlichen nur durch die Ausbildung im Bereich der Ladeöffnung und hier insbesondere der Gaszufuhrvorrichtung.

Der Erfindung liegt dabei die Erkenntnis zugrunde, dass eine beidseitig geschlossene Transportschleuse, wie sie in der US 6,467,285 B2 beschrieben ist, und ein vollständiger Austausch der Atmosphäre in der Transportschleuse nicht erforderlich ist, wenn Beeinträchtigungen der Bedingungen im Innenraum eines Klimaschranks verhindert werden sollen, sondern eine lediglich zur Außenseite hin verschließbare Ladeöffnung ausreicht, wenn man für eine geeignete Führung des Gasstroms im Bereich der Ladeöffnung sorgt.

Im erfindungsgemäßen Klimaschrank weist die Gaszufuhrvorrichtung daher wenigstens eine Gasausblasöffnung auf, die im Bereich der Ladeöffnung derart angeordnet ist, dass der Querschnitt der Ladeöffnung im Bereich der Gasausblasöffnungen bei Zufuhr des Gases von einem Gasvorhang abgedeckt wird. Der so erzeugte Gasvorhang schließt den Querschnitt der Ladeöffnung gegen das Eindringen von Umgebungsluft praktisch vollständig ab und verhindert so zuverlässig eine Beeinträchtigung des Klimas im Inneren des Klimaschrankes durch die Umgebungsatmosphäre. Damit wird gleichzeitig verhindert, dass mit der Umgebungsluft Feuchtigkeit in die Klimakammer gelangt und dort auskondensiert. Insofern ersetzt der Gasvorhang die innenseitige Transportschleusentür der US 6,467,285 B2 und vereinfacht so den Aufbau der Vorrichtung. Andererseits ist der Gasvorhang ohne weiteres von der Transportvorrichtung mit einem Objektträger passierbar, ohne dass eine längere Verweilzeit in der Ladeöffnung, deren beidseitiges Abschließen und ein vollständiger Gasaustausch in der Ladeöffnung erforderlich wären. Dadurch lässt sich der Transport beschleunigen.

Die wenigstens eine Gasausblasöffnung kann grundsätzlich irgendwo im Bereich der Ladeöffnung angeordnet sein, solange Anbringungsort und Öffnungsgröße sicherstellen, dass der Öffnungsquerschnitt der Ladeöffnung vollständig vom Gasfluss abgedeckt wird. Als Gasausblasöffnung kann beispielsweise eine Schlitzdüse dienen. Alternativ können zur Abdeckung des Öffnungsquerschnitts mehrere kleine Düsen eingesetzt werden.

Als zweckmäßig hat es sich erwiesen, die wenigstens eine Gasausblasöffnung im Bereich des nach außen weisenden Eingangs der Ladeöffnung anzuordnen, um so die Ladeöffnung möglichst insgesamt vor dem Eindringen von Umgebungsluft und dem Einschleppen von Feuchtigkeit zu schützen. Dies schließt aber nicht aus, dass Gasausblasöffnungen ausschließlich oder zusätzlich im innenseitigen Bereich der Ladeöffnung vorhanden sind.

Denkbar ist beispielsweise, die Gasausblasöffnungen außerhalb der Ladeöffnung im an das innere Ende der Öffnung angrenzenden Bereich so anzuordnen, dass die Ausströmöffnungen der Gasausblasöffnungen in Richtung auf deren äußere Öffnung hinweisen. Der Gasstrom durchströmt dann die Ladeöffnung vom inneren in Richtung auf das äußere Ende. Bevorzugt ist es jedoch, die Gasausblasöffnungen im Bereich der Ladeöffnung und genauer im Bereich der die Ladeöffnung definierenden Ladeöffnungs-Wände anzuordnen. Die Länge der Ladeöffnung wird dabei üblicherweise von der Dicke der Seitenwände bestimmt, welche den Innenraum des Klimaschranks umgeben. Es ist jedoch auch möglich, die Ladeöffnung über die Dicke der Seitenwände hinaus zu verlängern und die Ladeöffnungs-Wände in das Innere des Klimaschranks oder, bevorzugt, über das Äußere des Klimaschrankes hinaus vorstehen zu lassen.

Besonders zuverlässig lässt sich ein den Querschnitt der Ladeöffnung abdeckender Gasvorhang erzielen, wenn mehrere Gasausblasöffnungen zum Aufbau des Gasvorhangs verwendet werden. Beispielsweise können sich Gasausblasöffnungen auf entgegengesetzten Seiten der Ladeöffnung gegenüber liegen. Dies können zum Beispiel gegenüber liegende Seitenwände der Ladeöffnung sein. Diese Anordnung hat den Vorteil, dass bei Verwendung von Mikrotiterplatten oder ähnlichen Objektträger mit offen aufbewahrten flüssigen Proben Gas nicht direkt auf die Proben geblasen wird. Aber auch die Anordnung der Gasausblasöffnungen in Decken- und Bodenwand der Ladeöffnung - ausschließlich dort oder zusätzlich zu den Seitenwänden - ist möglich.

Die Anzahl der Gasausblasöffnungen hängt von der Größe der Ladeöffnung ab und sollte in jedem Fall ausreichen, um einen Gasstrom zu gewährleisten, der das Eindringen von Umgebungsluft aus dem Außenbereich des Klimaschranks in die Ladeöffnung verhindert. Dabei kann es zweckmäßig sein, in der Ladeöffnung mehrere Reihen von Gasausblasöffnungen anzuordnen. Eine hohe Zahl von Gasausblasöffnungen pro Fläche kann zum Beispiel durch gestaffelte Anordnung der Gasausblasöffnungen erzielt werden.

Sinnvoll kann es außerdem sein, die Strömungsrichtung der Gasausblasöffnungen gezielt einzustellen. Beispielsweise ist es möglich, wenigstens einen Teil der Gasausblasöffnungen schräg in Richtung auf das Äußere des Klimaschrankes hin auszurichten. Dadurch ergibt sich eine Gasströmung in Richtung auf den äußeren Eingang der Ladeöffnung und von dieser weg, und das Eindringen von Umgebungsluft wird zusätzlich erschwert. Es ist dafür nicht grundsätzlich erforderlich, sämtliche Gasausblasöffnungen in Richtung auf die Außenseite der Ladeöffnung hin auszurichten, jedoch ist dies gegenwärtig bevorzugt.

Bei Anbringung einer größeren Zahl von Gasausblasöffnungen in den Wänden der Ladeöffnung kann es für eine gleichmäßige und einfache Zuführung des Gases sinnvoll sein, die Ladeöffnung zumindest in den Bereichen, in denen die Öffnungen vorhanden sind, mit einer Kammer zu umgeben, die mit dem Gas gefüllt wird und von wo aus das Gas dann in die mit der Gassammelkammer in Verbindung stehenden Gasausblasöffnungen gelangt. So ist auch nur eine einzige, in die Gassammelkammer mündende Gaszuleitung nötig.

Zusätzlich oder anstelle der Anbringung des Gasausblasöffnungen in der Ladeöffnung kann wenigstens eine Gasausblasöffnung auch außerhalb der Ladeöffnung benachbart dem nach außen weisenden Eingang der Ladeöffnung vorgesehen sein. Wie schon im Fall der Gasausblasöffnungen in den Wänden der Ladeöffnung können auch in diesem Fall Gasausblasöffnungen beidseitig, ganz oder teilweise um die Ladeöffnung herum vorhanden sein. Aus den bereits vorstehen genannten Gründen kann es zweckmäßig sein, die Gasausblasöffnungen so anzubringen, dass eine unmittelbare Gaseinwirkung auf die Proben vermieden wird.

Ein weiterer Anbringungsort für wenigstens eine der Gasausblasöffnungen ist die die Ladeöffnung frontseitig verschließende Tür. Besonders bietet es sich bei Schiebetüren an, auf der Seite der Tür, die beim Öffnen über den Eingang der Ladeöffnung hinweg geschoben wird und im geöffneten Zustand benachbart zur Ladeöffnung zu liegen kommt, eine oder mehrere Gasausblasöffnungen anzubringen.

Wie schon im Fall der in der Ladeöffnung angeordneten Gasausblasöffnungen können die außen vor der Ladeöffnung vorhandenen Gasausblasöffnungen so orientiert sein, dass Gas schräg vom Klimaschrank weg ausströmt.

Als Gas kann grundsätzlich jedes im Hinblick auf die im Innenraum des Klimaschranks vorhandene Atmosphäre geeignete Gas verwendet werden. Bevorzugt werden im Wesentlichen wasserfreie Gase und/oder Inertgase (Schutzgase) eingesetzt, zum Beispiel getrocknete Luft, Stickstoff oder Kohlendioxid. Gegenwärtig ist Stickstoff bevorzugt. Dies hat den Vorteil, dass die in Laboratorien üblicherweise vorhandenen Stickstoffleitungen eingesetzt werden können, gegebenenfalls unter Einsatz eines Reduzierventils, das den Druck auf einen geeigneten Vordruck von beispielsweise 1 bar reduziert.

Um das Gas der Temperatur des Innenraums anzugleichen, kann die Gaszuleitung zur Temperierung des Gases durch den klimatisierten Innenraum und/oder durch den Wärmetauscherbereich der Vorrichtung geführt werden.

Außerdem ist es möglich, das Gas zusätzlich zur Beeinflussung der Atmosphäre im Innenraum des Klimaschranks zu verwenden. Sinnvoll ist dies zum Beispiel, wenn als Gas ein Inertgas wie Stickstoff verwendet wird und im Innenraum luftempfindliche Objekte gelagert werden. Einerseits ist es denkbar, von der Gaszuleitung für die Ladeöffnung eine Zuleitung für den Innenraum abzuzweigen und den Innenraum so mit Gas zu versorgen. Andererseits oder eventuell auch ergänzend kann eine Versorgung des Innenraums mit Gas über die Ladeöffnung erfolgen. Für eine ausreichende Gasversorgung des Innenraums kann es dann sinnvoll sein, die Gaszufuhr der Ladeöffnung über den Zeitraum hinaus zu verlängern, der zum Schutz des Klimaschrankes beim Be- und Entladen mit Objektträgern erforderlich wäre, und der Ladeöffnung Gas beispielsweise ununterbrochen zuzuführen.

Auf der anderen Seite kann es jedoch auch sinnvoll sein, die Gaszufuhr an das Öffnen der die Ladeöffnung verschließenden Tür zu koppeln. Hierfür ist dann im erfindungsgemäßen Klimaschrank eine Steuervorrichtung vorhanden, die ein Steuerventil in der Gaszufuhrvorrichtung derart steuert, dass das Öffnen und Schließen des Steuerventils - und damit das An- und Abschalten der Gaszufuhr in die Ladeöffnung - davon abhängt, ob die die Ladeöffnung verschließende Tür geöffnet oder geschlossen ist. Zweckmäßig wird das Steuerventil geöffnet, und Gas strömt ein, wenn die Tür vor der Ladeöffnung geöffnet wird. Alternativ kann die Gaszufuhr auch kurz vor dem Öffnen der Tür beginnen. Nach dem Schließen der Tür wird das Steuerventil ebenfalls geschlossen, und die Gaszufuhr wird beendet. Für diese Steuerfunktionen muss keine gesonderte Steuervorrichtung vorgesehen sein, sondern die Funktionen können in eine Steuervorrichtung integriert werden, die ohnehin bereits üblicherweise in einem Klimaschrank vorhanden ist und zum Beispiel die Transportvorrichtung zum Be- und Entladen der Objektträger steuert oder die Temperatur im Klimaschrank regelt. Zweckmäßig steuert diese Steuervorrichtung auch das automatische Öffnen und Schließen der Tür, welche die Ladeöffnung verschließt.

Die Gaszufuhrmenge in die Ladeöffnung hängt unter anderem von der Größe der Öffnung, der Anzahl der Gasausblasöffnungen, welche der Öffnung Gas zuführen, und den klimatischen Bedingungen im Innenraum des Klimaschrankes sowie in der Umgebung des Klimaschrankes ab. Die Gaszufuhr sollte ausreichend hoch eingestellt werden, um ein Eindringen von Umgebungsluft in die Ladeöffnung zu verhindern. Dabei kann es sinnvoll sein, die Steuervorrichtung so auszubilden, dass die Gaszufuhr in Abhängigkeit von den klimatischen Bedingungen im Innenraum einerseits und im Außenraum um den Klimaschrank andererseits geregelt wird. Besonders zweckmäßig ist es hier, die Temperaturdifferenz zwischen Innenraum und Außenumgebung des Klimaschranks zu beachten. Ist zum Beispiel die Temperatur im Innenraum des Klimaschranks deutlich niedriger als in der Außenumgebung des Klimaschranks, ist die Gefahr besonders groß, dass Feuchtigkeit in den Innenraum eindringt und dort kondensiert. Im Falle eines Klimakühlschranks kann es daher sinnvoll sein, die Gaszufuhr mit steigender Temperaturdifferenz zu erhöhen.

Die Erfindung soll nachfolgend anhand einer Zeichnung näher erläutert werden. Darin zeigen schematisch:
- Fig. 1: einen erfindungsgemäßen Klimaschrank im Querschnitt;
- Fig. 2: eine Draufsicht auf die Ladeöffnung des in Fig. 1 gezeigten Klimaschrankes vom Äußeren des Klimaschrankes her gesehen;
- Fig. 3: einen Querschnitt durch eine Seitenwand der Ladeöffnung entlang der Linie A-A in Fig. 2;
- Fig. 4: ein anderes Beispiel eines erfindungsgemäßen Klimaschrankes im Querschnitt im Bereich der Ladeöffnung;
- Fig. 5: eine Draufsicht auf die Ladeöffnung gemäß Fig. 4 und
- Fig. 6: eine alternative Ausführungsform zur Ausführung gemäß Fig. 5, ebenfalls in Draufsicht.

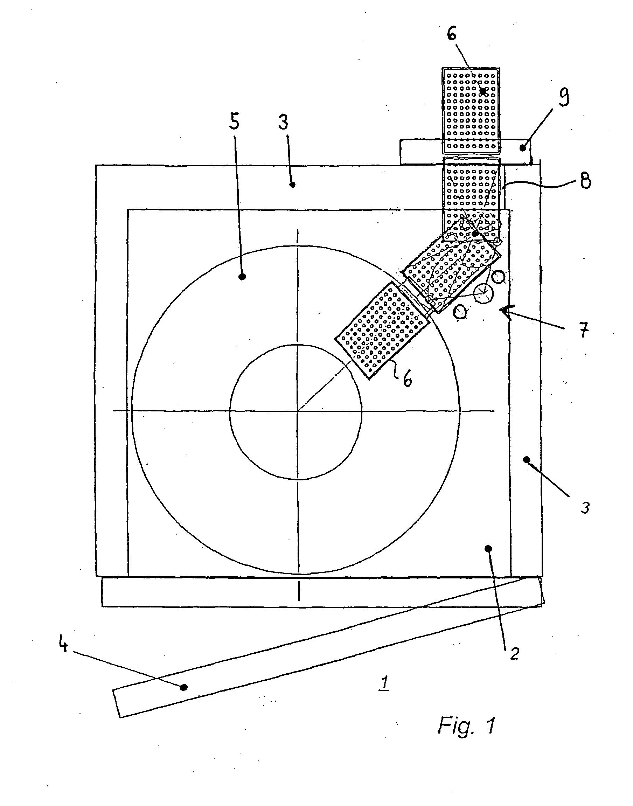

Der in Fig. 1 gezeigte Klimaschrank 1 weist einen Innenraum 2 auf, welcher von Seitenwänden 3 umgeben und mit einer manuell zu öffnenden Tür 4 verschließbar ist. Diese nachfolgend als Haupttür bezeichnete Tür 4, die sowohl im geschlossenen als auch im halb geöffneten Zustand gezeigt ist, nimmt von ihrer Größe her im Wesentlichen die gesamte Vorderseite des Klimaschranks 1 ein. Im Innenraum 2 ist ein Haltegestell in Form eines drehbar gelagerten Karussells 5 aufgestellt, in welchem eine Vielzahl von Objektträgern gelagert werden kann. Für das Be- und Entladen der Objektträger 6 ist im rückwärtigen Bereich des Klimaschranks 1 (rechts oben in Fig. 1) eine Transportvorrichtung 7 vorhanden. Der Transport erfolgt durch eine Ladeöffnung 8 in der Rückwand des Klimaschranks 1. Die Größe der Ladeöffnung 8 ist der Größe der Objektträger 6 angepasst und gerade nur so groß, dass die Objektträger 6 mit der Transportvorrichtung 7 durch die Ladeöffnung 8 transportiert werden können. Die äußere Seite der Ladeöffnung 8 ist mit einer automatisch öffnenden und schließenden Tür 9 dicht verschließbar. Öffnen und Schließen der Tür 9 erfolgen koordiniert mit der Transportvorrichtung 7 über eine Steuervorrichtung, die hier nicht dargestellt ist. Verschiedene Transportetappen eines einzelnen Objektträgers 6 von außerhalb des Klimaschranks bis in das Karussell 5 sind durch Wiedergabe von vier Transportstadien des Objektträgers angedeutet. Der dargestellte Klimaschrank 1 entspricht im Wesentlichen demjenigen, der in der WO 98/05753 A1 beschrieben ist. Er unterscheidet sich von diesem jedoch in der Ausgestaltung der Ladeöffnung 8.

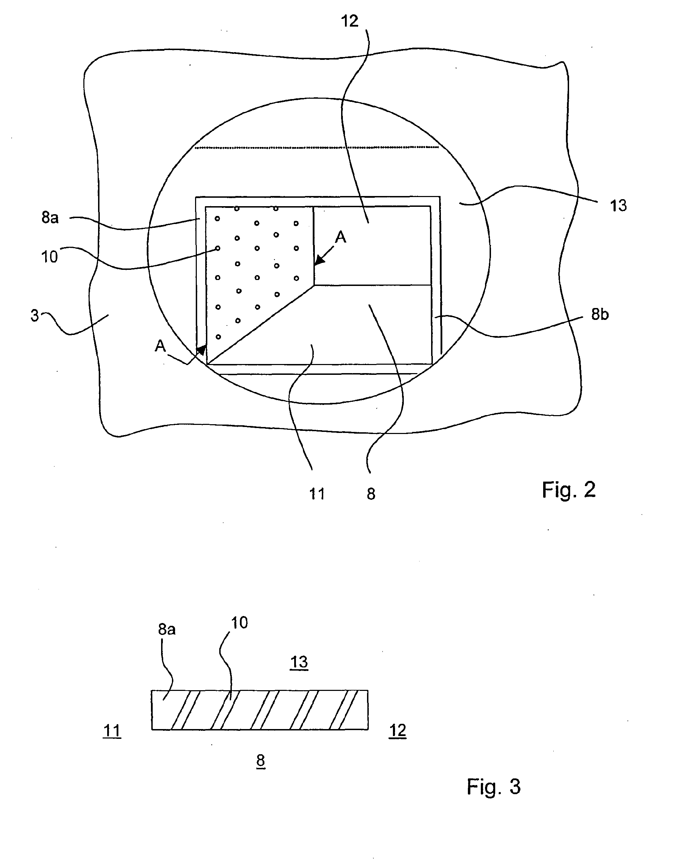

Im gezeigten Beispiel des erfindungsgemäßen Klimaschranks sind in der Ladeöffnung 8 Gasausblasöffnungen angeordnet, welche der Ladeöffnung ein Gas, hier Stickstoff, zuführen. Die Gasausblasöffnungen sind in Fig. 1 nicht wiedergegeben, jedoch in Fig. 2 dargestellt und mit Bezugszeichen 10 bezeichnet.

Fig. 2 ist eine Draufsicht auf die Ladeöffnung 8 und die rückwärtige (in Fig. 1 obere) Wand 3 vom Äußeren des Klimaschrankes 1 her gesehen. Die den äußeren Eingang 11 der Ladeöffnung 8 verschließende Tür 9 ist in Fig. 2 nicht dargestellt. Ebenso sind die Transportvorrichtung 7 und Objektträger 6 der Übersichtlichkeit halber weggelassen. Letztere entsprechen dem Stand der Technik.

Im Unterschied zum Stand der Technik sind im Inneren der Ladeöffnung 8 jedoch Gasausblasöffnungen 10 vorgesehen, um einen den Querschnitt der Ladeöffnung 8 abdeckenden Gasvorhang zu erzeugen. Wie im linken Teil der Fig. 2 zu sehen, sind im Bereich der linken Seitenwand 8a gleichmäßig über deren Fläche verteilt 5 Reihen zu je 5 Gasausblasöffnungen vorhanden. Diese Gasausblasöffnungen 10 gehen durch die Seitenwand 8a hindurch und münden in eine die Ladeöffnung 8 umgebende und durch die punktierte Linie verdeutlichte Gassammelkammer 13, die über einen Gasanschluss mit Gas versorgt wird. Diese Gassammelkammer 13 dient dem Aufbau eines Gasvordrucks und der gleichmäßigen Verteilung des Gases.

Auf der der Wand gegenüberliegenden Seite sind in der Wand 8b ebenfalls Gasausblasöffnungen wie in der Wand 8a vorhanden. Durch die gleichmäßige Verteilung der Gasausblasöffnungen 10 im Bereich der die Ladeöffnung 8 umgebenden Ladeöffnungs-Seitenwände 8a und 8b kann über die gesamte Länge der Ladeöffnung 8 vom inneren Eingang 12 auf der Seite des Innenraums 2 bis zum äußeren Eingang 11 auf der Außenseite des Klimaschranks ein breiter Gasvorhang erzeugt werden, der den gesamten Öffnungsquerschnitt der Ladeöffnung 8 abdeckt. Dadurch kann das Eindringen von Umgebungsluft in den Innenraum 2 zuverlässig verhindert werden, ohne dass eine den inneren Eingang 12 verschließende Tür nötig wäre, wie dies in der US 6,467,285 B2 beschrieben ist.

Fig. 3 zeigt eine bevorzugte Anordnung der Gasausblasöffnungen 10 in einer der Wände der Ladeöffnung 8, hier beispielhaft der Seitenwand 8a. Der Querschnitt durch die Wand 8a entlang der Linie A-A verdeutlicht, dass alle Gasausblasöffnungen schräg mit ihren in die Ladeöffnung 8 weisenden Öffnungen in Richtung auf die äußere Öffnung 11 hin verlaufen. Dadurch ergibt sich eine Gasströmung, welche aus der Ladeöffnung 8 in Richtung auf die äußere Öffnung 11 hin gerichtet ist. Auf diese Weise kann ein Eindringen von Umgebungsluft durch die Öffnung 11 in die Ladeöffnung 8 besonders sicher verhindert werden.

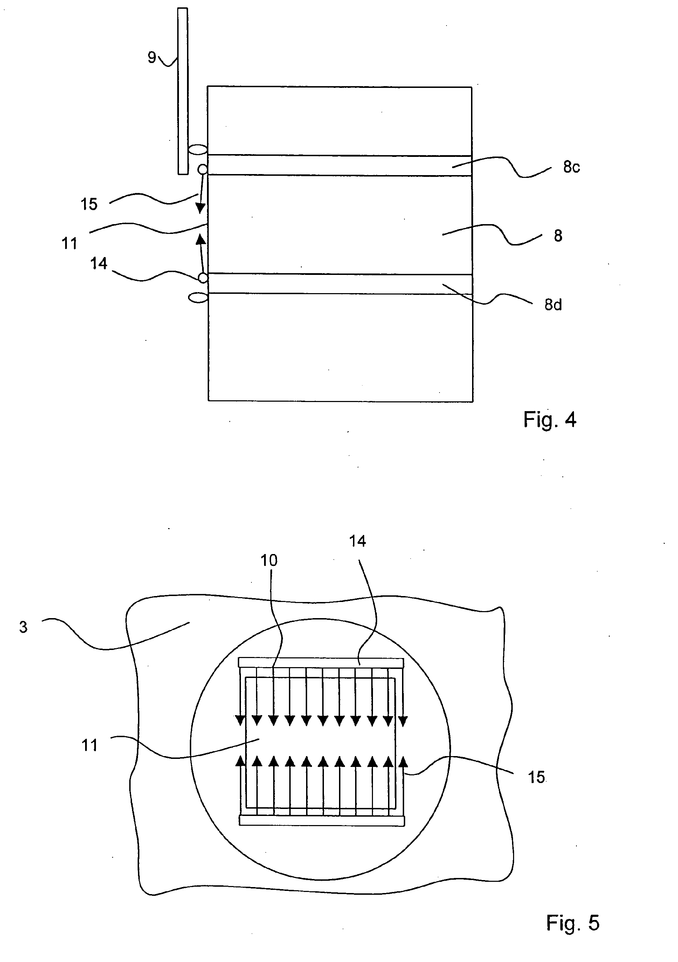

Fig. 4 veranschaulicht eine weitere Ausführungsform der Erfindung, in der ein Gasvorhang auf der Außenseite der Ladeöffnung 8 erzeugt wird. Hierfür sind zwei Gasverteilerrohre 14 ober- und unterhalb des äußeren Eingangs 11, hier vor der oberen Wand 8c und der unteren Wand 8d der Ladeöffnung 8, angeordnet. Die Gasverteilerrohre weisen gleichmäßig über ihre Länge verteilt Gasausblasöffnungen 10 auf, die jeweils in Richtung auf den Eingang 11 hin ausgerichtet sind (vgl. Fig. 5) und zwar hier derart, dass der Gasstrom 15 leicht vom Eingang 11 weg nach außen gerichtet ist.

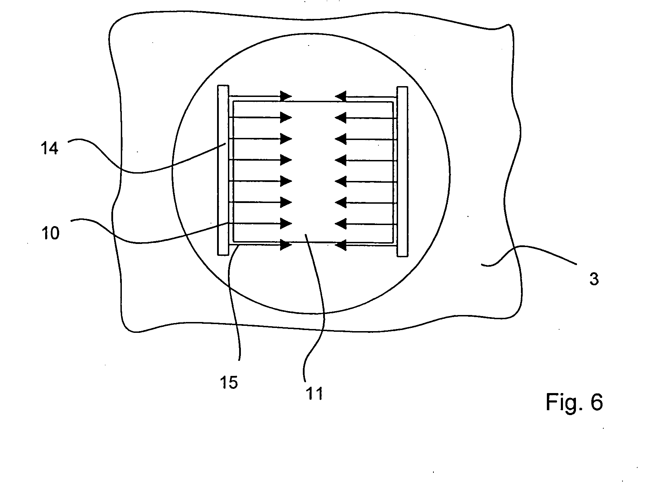

Fig. 6 zeigt eine Alternative zur Anordnung der Gasverteilerrohre in Fig. 5. Hier sind die Gasverteilerrohre 14 rechts und links des äußeren Eingangs 11 angeordnet. Dies hat den Vorteil, dass ein Objektträger beim Transport nicht von oben vom Gasstrom 15 getroffen wird, was bei nach oben offenen Objektträgern wie beispielsweise Mikrotiterplatten mit offen gelagerten Proben nachteilig sein kann.
Eine Kombination beider Ausführungsformen nach Fig. 5 und Fig. 6 zu einer allseitig von Gasausblasöffnungen umgebenen Ladeöffnung ist selbstverständlich ebenfalls möglich.

## Patentansprüche

1. Klimaschrank mit einem Innenraum zur Aufnahme von Objektträgern und mit einer Transportvorrichtung zum Be- und/oder Entladen des Innenraums mit Objektträgern und einer Ladeöffnung für das Durchtransportieren der Objektträger während des Be- und/oder Entladens, welche in einer den Innenraum umgebenden Seitenwand des Klimaschranks angeordnet, in ihrer Größe den Abmessungen der Objektträger angepasst und mit einer auf der Außenseite der Ladeöffnung befindlichen Tür dicht verschließbar ist, und in welchem eine Gaszufuhrvorrichtung vorhanden ist, um im Bereich der Ladeöffnung einen Gasstrom zuzuführen,
**dadurch gekennzeichnet,**
**dass** die Gaszufuhrvorrichtung wenigstens eine Gasausblasöffnung aufweist, die im Bereich der Ladeöffnung derart angeordnet ist, dass der Querschnitt der Ladeöffnung im Bereich der Gasausblasöffnungen bei Zufuhr des Gases von einem Gasvorhang abgedeckt wird.

2. Klimaschrank gemäß Anspruch 1,
**dadurch gekennzeichnet,**
**dass** die wenigstens eine Gasausblasöffnung im Bereich des nach außen weisenden Eingangs der Ladeöffnung angeordnet ist.

3. Klimaschrank gemäß Anspruch 1 oder 2,
**dadurch gekennzeichnet,**
**dass** die wenigstens eine Gasausblasöffnung im Bereich wenigstens einer der die Ladeöffnung definierenden Ladeöffnungs-Wände in die Ladeöffnung einmündet.

4. Klimaschrank gemäß Anspruch 3,
**dadurch gekennzeichnet,**
**dass** Gasausblasöffnungen im Bereich mehrerer, insbesondere gegenüber liegender, oder aller Ladeöffnungs-Wände vorhanden sind.

5. Klimaschrank gemäß Anspruch 3 oder 4,
**dadurch gekennzeichnet,**
**dass** mehrere Reihen von Gasausblasöffnungen hintereinander angeordnet sind.

6. Klimaschrank gemäß einem der Ansprüche 3 bis 5,
**dadurch gekennzeichnet,**
**dass** wenigstens ein Teil der Gasausblasöffnungen schräg in Richtung auf das Äußere des Klimaschrankes hin ausgerichtet ist.

7. Klimaschrank gemäß einem der Ansprüche 3 bis 6,
**dadurch gekennzeichnet,**
**dass** die Ladeöffnung zumindest in den Bereichen, in welche Gasausblasöffnungen einmünden, von einer Gassammelkammer umgeben ist, die mit den Gasausblasöffnungen kommuniziert und die an eine Gaszuleitung angeschlossen ist.

8. Klimaschrank gemäß einem der Ansprüche 1 bis 7,
**dadurch gekennzeichnet,**
**dass** das Gas ein im Wesentlichen wasserfreies Gas und/oder ein Inertgas, insbesondere Stickstoff, ist.

9. Klimaschrank gemäß einem der Ansprüche 1 bis 8,
**dadurch gekennzeichnet,**
**dass** die für die Zuleitung des Gases zu der wenigstens einen Gasausblasöffnung verwendete wenigstens eine Gasleitung zur Temperierung durch den Innenraum und/oder den Wärmetauscherbereich des Klimaschrankes geführt wird.

10. Klimaschrank gemäß einem der Ansprüche 1 bis 9,
**dadurch gekennzeichnet,**
**dass** die wenigstens eine Gasausblasöffnung außerhalb der Ladeöffnung benachbart dem nach außen weisenden Eingang der Ladeöffnung angeordnet ist.

11. Klimaschrank gemäß Anspruch 10,
**dadurch gekennzeichnet,**
**dass** Gasausblasöffnungen entlang zumindest zweier sich gegenüber liegender Seiten am Eingang der Ladeöffnung angeordnet sind.

12. Klimaschrank gemäß einem der Ansprüche 1 bis 11,
**dadurch gekennzeichnet,**
**dass** wenigstens eine Gasausblasöffnung in die die Ladeöffnung verschließende Tür integriert ist.

13. Klimaschrank gemäß einem der Ansprüche 9 bis 12,
**dadurch gekennzeichnet,**
**dass** die wenigstens eine Gasausblasöffnung schräg in Richtung vom Klimaschrank weg ausgerichtet ist.

14. Klimaschrank gemäß einem der Ansprüche 1 bis 13,
**dadurch gekennzeichnet,**
**dass** er eine Steuervorrichtung aufweist, welche ein Steuerventil in der Gaszufuhrvorrichtung derart steuert, dass die Gaszufuhr an die Öffnung der die Ladeöffnung verschließenden Tür gekoppelt ist.

15. Klimaschrank gemäß Anspruch 14,
**dadurch gekennzeichnet,**
**dass** die Steuervorrichtung derart ausgebildet ist, dass die Gaszufuhr beim oder kurz vor dem Öffnen der Tür beginnt und nach dem Schließen der Tür beendet wird.

16. Klimaschrank gemäß einem der Ansprüche 1 bis 15,
**dadurch gekennzeichnet,**
**dass** die Tür eine automatisch öffnende und schließende Tür ist.

17. Klimaschrank gemäß einem der Ansprüche 1 bis 16,
**dadurch gekennzeichnet,**
**dass** die Steuervorrichtung ausgebildet ist, um die Gaszufuhr in Abhängigkeit der klimatischen Bedingungen im Innenraum und außerhalb des Klimaschranks und insbesondere in Abhängigkeit von der Temperaturdifferenz zwischen Innen- und Außenraum zu regeln.

18. Klimaschrank gemäß einem der Ansprüche 1 bis 17, nämlich Klimakühlschrank.
